(19) Europäisches Patentamt
European Patent Office
Office européen des brevets

(11) Veröffentlichungsnummer: **0 203 152**
**B1**

(12) # EUROPÄISCHE PATENTSCHRIFT

(45) Veröffentlichungstag der Patentschrift: **22.06.88**

(51) Int. Cl.⁴: **C 07 C 121/46**, C 07 C 120/00, C 11 B 9/00, A 61 K 7/46

(21) Anmeldenummer: **85906037.8**

(22) Anmeldetag: **27.11.85**

(86) Internationale Anmeldenummer: **PCT/DE 85/00499**

(87) Internationale Veröffentlichungsnummer: **WO 86/03196 (05.06.86** Gazette **86/12)**

(54) **METHYLSUBSTITUIERTE BICYCLO AD2.2.1 BDHEPTAN-NITRILE, VERFAHREN ZUR HERSTELLUNG DIESER NITRILE UND DEREN VERWENDUNG ALS RIECHSTOFFE.**

(30) Priorität: **29.11.84 DE 3443536**

(43) Veröffentlichungstag der Anmeldung: **03.12.86 Patentblatt 86/49**

(45) Bekanntmachung des Hinweises auf die Patenterteilung: **22.06.88 Patentblatt 88/25**

(84) Benannte Vertragsstaaten: **CH DE FR GB LI NL**

(56) Entgegenhaltungen:
**US - A - 4 390 464**

**Chemical Abstracts, vol. 96, Nr. 23, 7 June 1982, Columbus, Ohio (US) - M. Lajunen et al.: Acid-catalyzed hydrolyses of bridged bi- and tricyclic compounds. Part XIX. Kinetics of hydration of some 2-cyanonorbornanes, page 590, abstract 198769f**

(73) Patentinhaber: **Dragoco Gerberding & Co. GmbH, Dragocostrasse 1, D-3450 Holzminden (DE)**

(72) Erfinder: **BRUNKE, Ernst-Joachim, Holbeinstrasse 6, D-3450 Holzminden (DE)**
Erfinder: **STRUWE, Hartmut, Am Feldberg 25, D-3470 Höxter 1 (DE)**

(74) Vertreter: **Eikenberg & Brümmerstedt Patentanwälte, Schackstrasse 1, D-3000 Hannover 1 (DE)**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



ACTORUM AG

**Beschreibung**

Die Erfindung betrifft als neue Verbindungen die Nitrile des dimethylsubstituierten Bicyclo-[2.2.1]heptans mit der allgemeinen Formel A

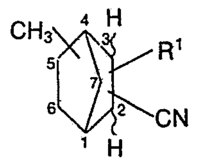

$R^1 = CH_3$

A

worin die Methylgruppe an C–4 oder C–5 steht, der Rest $R^1$ an C–2 und die Nitrilgruppe an C–3 oder alternativ der Rest $R^1$ an C–3 und die Nitrilgruppe an C–2 stehen, zwischen C–5 und C–6 eine Einfachbindung vorliegt und die geschlängelten Linien stereoisomere Formen bedeuten. Die Erfindung betrifft weiterhin ein vorteilhaftes Verfahren zur Herstellung der neuen Verbindungen gemäss der allgemeinen Formel A sowie die Verwendung dieser neuen Verbindungen als Riechstoffe bzw. als Bestandteile von Parfümölen für kosmetische oder technische Konsumgüter.

Es sind Derivate des methyl- und dimethylsubstituierten Bicyclo[2.2.1]heptans (Methyl- und Dimethylnorbornan-Derivate) sowie des strukturähnlichen methyl- und dimethylsubstituierten Bicyclo[2.2.1]hept-5-ens (Methyl- und Dimethylnorbornen-Derivate) bekannt, die als Riech- und Aromastoffe geeignet sind. So beschreibt die US-PS 4 326 998 (Lemarczyk et al.) den Methylnorbornen-Aldehyd 4 und den Dimethylnorbornen-Aldehyd 5, die durch Diensynthese (Diels-Alder-Reaktion) aus Methylcyclopentadien 1 mit Acrolein 2 bzw. Crotonaldehyd 3 erhalten werden, sowie durch die entsprechenden gesättigten Aldehyde 6 und 7, die durch Hydrierung aus den Aldehyden 5 bzw. 6 entstehen. Diese Aldehyde 4 bis 7 besitzen grüne, frisch geschnittenem Gras ähnliche und minzige Duftnoten mit fruchtigen (apfelartigen) und krautigen Nebennoten.

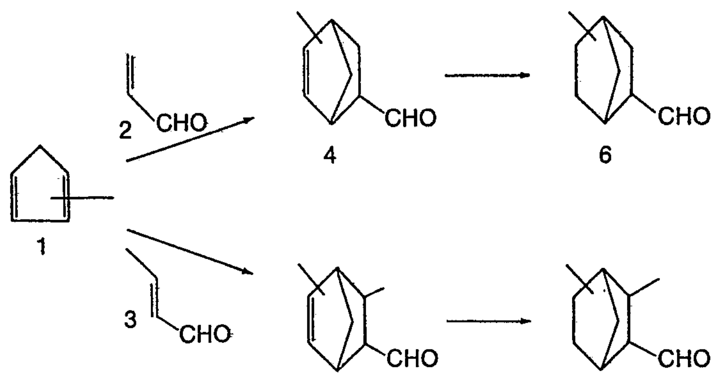

2 4 6 1 3 5 7

Weiterhin beschreibt die US-PS 4 390 464 (Klemarczyk et al.) das Methylnorbornen-Nitril 9, das durch Diensynthese aus

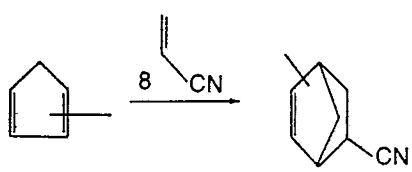

8

1 9

Methylcyclopentadien 1 mit Acrylnitril 8 gebildet wird und einen blumig-fruchtigen Duft (Jasmin-Typ) besitzt.

Nicht als Riech- und Aromastoff, aber als Verbindung ist im übrigen auch das dem Nitril 9 entsprechende Methylnorbornannitril 12 bekannt. Dieses Nitril 12 ist von Lajunen und Tallgren,

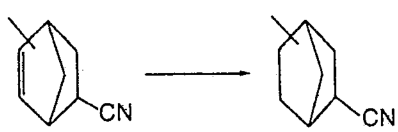

9 12

Finn. Chem. Lett. 1981, 106–109 im Rahmen einer reaktionskinetischen Untersuchung beschrieben und wurde durch Hydrierung des Nitrils 9 in Aceton mit einem Palladium-Katalysator hergestellt. Irgendwelche olfaktorischen Eigenschaften der Nitrile 9 und 12 sind dabei nicht erwähnt.

Generell besteht ein ständiger Bedarf an synthetischen Riechstoffen, die sich günstig und mit gleichbleibender Qualität herstellen lassen, bei längerer Lagerung auch im Kontakt mit anderen Stoffen stabil bleiben und erwünschte olfaktorische Eigenschaften haben, d.h. angenehme, möglichst naturnahe Duftnoten von ausreichender Intensität aufweisen und in der Lage sind, den Duft von kosmetischen oder technischen Konsumgütern vorteilhaft zu beeinflussen. Das Auffinden solcher Riechstoffe, die sämtliche dieser Bedingungen erfüllen, hat sich als verhältnismässig aufwendig erwiesen und erfordert umfangreiche Untersuchungen insbesondere dann, wenn interessante neuartige Duftnoten angestrebt werden. Erschwerend kommt in diesem Zusammenhang dabei die Tatsache hinzu, dass sich die Qualität der einzelnen Duftnoten nicht quantitativ beschreiben lässt, und dass auch weder der Mechanismus der Duftentwicklung noch der Zusammenhang zwi-

schen der Duftentwicklung und der chemischen Struktur von Riechstoffen hinreichend systematisch erforscht sind. Mitunter können bereits geringfügige Änderungen im Aufbau bekannter Riechstoffe starke Änderungen der olfaktorischen Eigenschaften sowohl in positiver als auch in negativer Richtung ergeben, und mitunter wirken sich solche Änderungen praktisch überhaupt nicht aus.

Über die erfindungsgemässen Dimethylnorbornan-Nitrile gemäss der allgemeinen Formel A war bislang noch nichts bekannt. Diese erfindungsgemässen Nitrile A unterscheiden sich von den bekannten Nitrilen 9 und 12 strukturell darin, dass das Ringsystem keine Doppelbindung aufweist und dass der Rest $R^1$ eine Methylgruppe ist. Überraschenderweise führt der Strukturunterschied auch zu ganz anderen Geruchseigenschaften. Während das bekannte ungesättigte methylsubstituierte Nitril 9 dem Geruchs-Grundtyp «blumig-fruchtig» zuzuordnen ist (das entsprechende ungesättigte dimethylsubstituierte Nitril ist nicht bekannt und das gesättigte methylsubstituierte Nitril 12 kann nicht zu einem olfaktorischen Vergleich herangezogen werden), gehören die erfindungsgemässen gesättigten Nitrile A in den davon stark unterschiedlichen Geruchs-Grundtyp «krautig-würzig», der stark gefragt und sehr erwünscht ist. Im übrigen erfüllen die erfindungsgemässen Nitrile A auch alle übrigen Anforderungen an einen Riechstoff. Sie sind günstig mit gleichbleibender Qualität herstellbar und stehr stabil. Ausserdem sind die Duftnoten sehr intensiv und so beschaffen, dass bereits ein Zusatz verhältnismässig geringer Mengen der erfindungsgemässen Nitrile A z.B. zu Parfümölen eine deutlich verbessernde Wirkung hat. Insgesamt stellen die erfindungsgemässen Nitrile A damit sehr wertvolle Riechstoffe dar. Ausserdem sind sie aber auch gut als Aromastoffe bzw. als Bestandteile von Aromakonzentraten für Nahrungs- und Genussmittel oder Tiernahrungsmittel geeignet.

Eine Herstellung der erfindungsgemässen Nitrile A durch Hydrierung (analog der Herstellung des gesättigten Nitrils 12 aus dem ungesättigten Nitril 9) ist nur mit schlechter Ausbeute möglich, weil bei der Hydrierung auch die Nitrilgruppe reagieren kann. Mit der Erfindung wird deshalb ein

7 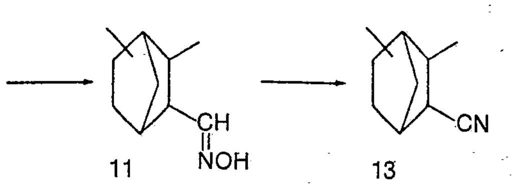

11 13

zweistufiges, gut reproduzierbares Verfahren vorgeschlagen, welches von dem gesättigten Adlehyd 7 ausgeht und darin besteht, dass zunächst aus diesem Aldehyd 7 in üblicher Weise (bevorzugt mit Hydroxylamin-Salzen) das entsprechende Aldoxim 11 dargestellt wird und anschliessend das Aldoxim 11 durch Wasserentzug (bevorzugt mit Säureanhydriden) in das gewünschte Nitril 13 überführt wird.

Das in die Diels-Alder-Reaktion zur Herstellung des Aldehyds 5 eingesetzte Methylcyclopentadien 1 ist ein Gemisch der drei Doppelbindungsisomere 1a, 1b und 1c, wobei im Gleichgewicht etwa 44,5% des C-1-Isomeren 1a, 54,5% des C-2-Isomeren 1b und nur ca. 1% des C-5-Isomeren 1c vorliegen (W.T. Ford, J. Org. Chem., 25, 3979 (1971); S. McLean und P. Haynes, Tetrahedron, 21, 2323 (1965)). Entsprechend bildet sich bei der Diels-Alder-Reaktion eines Gleichgewichts-Gemisches von 1 mit Crotonaldehyd 3 ein Aldehydgemisch 5 aus drei Konstitutionsisomeren des Typs a, b und c, indem aus dem Isomeren 1a unter Einhaltung des ortho-Prinzips der Aldehyd 5a, aus dem Isomeren 1b unter Einhaltung des para-Prinzips der Aldehyd 5b und aus dem Isomeren 1c der Aldehyd 5c entstehen. Die Isomeren der Typen a und b bilden dabei die Hauptisomeren, während das Isomere des Typs c nur untergeordnete Bedeutung hat. Bei allen drei Konstitutionsisomeren der Typen a, b und c kommt noch eine exo/endo-Stereoisomerie hinzu (die grafisch durch eine geschlängelte Linie angedeutet ist), wobei sich bei der thermischen Diels-Alder-Reaktion bevorzugt die endo-Isomeren ergeben. Diese Isomerieverhältnisse bleiben bei den aus dem Aldehyd 5 abgeleiteten Folgeprodukten 7, 11 und 13

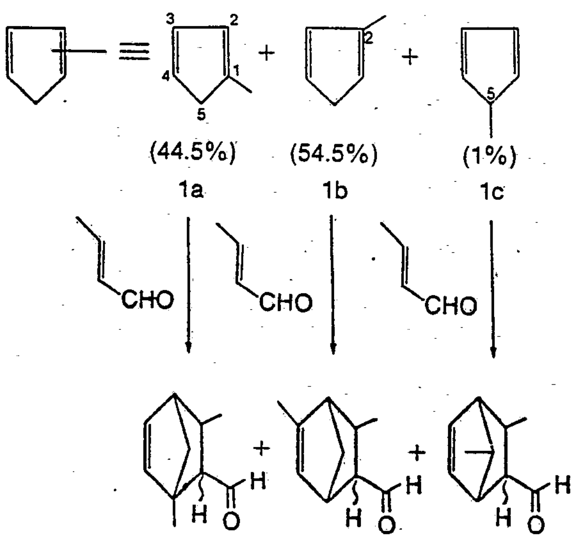

(44.5%) (54.5%) (1%)

1a 1b 1c

5 Typ a Typ b Typ c

im wesentlichen erhalten und sind in der allgemeinen Formel A berücksichtigt, nicht aber in der vereinfachten Formel 13.

Somit stellt das Nitril 13 ein Isomerengemisch entsprechend der allgemeinen Formel A dar. Dieses Isomerengemisch besitzt eine frische krautigwürzige Note, die an Rosmarin und Tabak erinnert. Solche Geruchsnoten sind für Norbornan-Derivate ungewöhnlich.

Die nachfolgenden Ausführungsbeispiele sollen die Erfindung erläutern, ohne sie einzuschränken.

Beispiel 1

Dimethylsubstituiertes 2-Cyano-bicyclo[2.2.1]heptan 13

a) Darstellung des Aldehyds 5

Zu einer Lösung von 9 g Hydrochinon in 1074 g (15.3 mol) Crotonaldehyd und 175 g n-Hexan wurde bei Siedetemperatur unter Rühren innerhalb von 3 Stunden 1215 g (15.2 mol) frisch destilliertes Methylcyclopentadien hinzugetropft. Nach 2 Stunden Rühren bei Siedetemperatur (Sumpftemp. ca. 110 °C) wurde über eine 40 cm-Vigreux-Kolonne destilliert. Man erhielt 1983 g des Aldehyds 5 (Isomerengemisch) als helles Öl.

Gaschromatogramm (30 m DB WAX-3ON, 40–240 °C, Temperaturprogramm: 4 °C/min): $t_r$[min] (%) = 13.4 (17.2), 13.5 (9.5), 13.0 (1.5), 14.4 (3.0), 14.6 (2.0), 15.2 (24.2), 15.3 (11.6), 15.5 (6.5).

b) Selektive Hydrierung des Aldehyds 5

Eine Lösung von 1465 (8.1 mol) des Aldehyds 5 (Isomerengemisch aus Beispiel 1a) in 800 ml Methanol wurde mit 20 g Raney-Nickel versetzt und in einer Wasserstoffatmosphäre bei 40 bar und 40 °C gerührt; theoret. Wasserstoffaufnahme: 86 bar, prak. Wasserstoffaufnahme: 90.5 bar. Nach Filtrieren, Einengen unter vermindertem Druck und einfacher Destillation ergaben sich 1420 g roher Aldehyd 7.

Gaschromatogramm: Abb. 1

Massenspektrum (Hauptpeak): m/z (%) = 152 (2, $M^+$), 137 (2), 119 (12), 108 (66), 93 (11), 81 (100), 71 (14), 67 (16).

Summenformel: $C_{10}H_{16}O$; Molgewicht: 152

c) Darstellung des Aldoxims 11

Zu einer Lösung von 961 g (5.93 mol) Hydroxylaminhydrogensulfat in 2,14 l Wasser wurde unter Rühren 926 g Natronlauge (50%ig) zugegeben, und dann wurde bei 20–30 °C 1466 g roher Aldehyd 7 (nach Beispiel 1b) innerhalb von 1.5 Stunden hinzugetropft. Nach 4 Stunden Rühren bei Raumtemperatur wurde mit 2.0 l Wasser verdünnt und in üblicher Weise aufgearbeitet (Extraktion mit Benzin). Das Rohprodukt (1412 g hellgelbes Öl) wurde über eine 60 cm Glasfüllkörperkolonne destilliert und ergab 894 g (72%) Aldoxim 11.

Siedepunkt: Kp (1.5 mbar) = 108–116 °C; Dichte: $D_{4°}^{20°}$ = 1.0104;

Brechungsindex: $n_D^{20°}$ = 1.5002.

Massenspektrum (Hauptpeak): m/z (%) = 157 (1, $M^+$), 150 (49), 108 (22), 107 (37), 86 (100), 81 (33), 79 (22), 70 (22), 67 (32), 55 (29), 41 (22).

Summenformel: $C_{10}H_{17}NO$; Molgewicht: 167.

d) Dehydratisierung des Aldoxims 11

Bei Siedetemperatur wurden unter Rühren zu 1039 g (10.1 mol) Acetanhydrid in 20 min 894 g (5.35 mol) des Oxims 11 (dargestellt nach Beispiel 1c) hinzugetropft. Nach 4 Stunden Rühren bei Siedetemperatur wurde Essigsäure unter vermindertem Druck abdestilliert. Vorsichtige Zugabe von Wasser und übliches Aufarbeiten ergaben 790 g Rohprodukt. Durch Destillation über eine 70 m Strahlfüllkörperkolonne erhielt man 652 g des Nitrils 13 als Isomerengemisch.

Siedepunkt: Kp (0.6 mbar) = 55–57 °C; Dichte: $D_{4°}^{20°}$ = 0.9458;

Brechungsindex: $n_D^{20°}$ = 1.4697.

Gaschromatogramm (50 m, FFAP, 60–220 °C, Temperaturprogramm 4 °C/min): $r_R$ [min] (%) = 19.6 (25.2), 20,8 (17.3), 22.8 (46.8), 23.1 (4.5), 23.8 (7.3), 24.4 (3.4).

e) Charakterisierung des Nitrils 13

Durch Destillation einer analytischen Probe des Nitrils 13 (gemäss Beispiel 1d) über eine Drehbandkolonne wurden die beiden Hauptisomere ($t_R$ = 19.6 bzw. 22.8 min) isoliert. Die weitere Untersuchung ergab:

2-Cyano-3,4-dimethyl-bicyclo[2.2.1]heptan

Gaschromatogramm: $t_R$ = 19.6 min

Infrarotspektrum: 2230 $cm^{-1}$ (Nitril)

$^1$H-NMR-Spektrum: Abb. 2

Massenspektrum: m/z (%) = 149 (1, $M^+$), 134 (22), 107 (19), 106 (65), 96 (19), 94 (16), 82 (24), 81 (100), 79 (18).

Summenformel: $C_{16}H_{15}N$; Molgewicht: 149.

2-Cyano-3,5-dimethyl-bicyclo[2.2.1]heptan

Gaschromatogramm: $t_R$ = 22.8 min

Infrarotspektrum: 2230 $cm^{-1}$ (Nitril)

$^1$H-NMR-Spektrum: Abb. 3

Massenspektrum: m/z (%) = 149 (2, $M^+$), 134 (25), 108 (23), 106 (37), 94 (27), 83 (48), 82 (100), 81 (75), 80 (97), 79 (51), 67 (74), 55 (38), 41 (26).

Summenformel: $C_{10}H_{15}N$; Molgewicht: 149.

Beispiel 2

Verwendung des Nitrils 13 in einer Parfümbase

Ausgegangen wurde von einer Parfümbase krautigen Typs mit der Zusammensetzung (GT = Gewichtsteile):

| | | |
|---|---|---|
| 190 | GT | Phyllanton-N |
| 100 | GT | Methyldihydrojasmonat |
| 100 | GT | Bergamotte-Öl |
| 90 | GT | Timberol |
| 90 | GT | Ysop-Öl |
| 80 | GT | 3-Phenyl-1,1-dimethyl-propanol (Muguet-Alkohol) |
| 30 | GT | Allyljonon |
| 10 | GT | Brahmanol |
| 10 | GT | 1,5-Dimethyl-8-hydroximino-bicyclo[3.2.1]octan (10%ig in Dipropylenglykol) |
| 10 | GT | Ambroxan, 1%ig in Dipropylenglykol |
| 700 | GT | |

Diese Parfümbase hat einen ausgewogenen frischen krautig-süssen und holzigen Duftkomplex. Durch Zugabe von 10 GT des Nitrils 13 wird eine bitter-grüne Geruchsnote bewirkt, die als ausgesprochen angenehm empfunden wird, und die Komposition erinnert dann an Muskateller Salbei-Öl.

**Patentansprüche**

1. Dimethylsubstituierte Bicyclo[2.2.1]heptan-Nitrile der allgemeinen Formel A

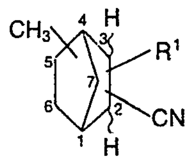

$R^1 = CH_3$

A

worin

die Methylgruppe an C–4 oder C–5 steht, der Rest $R^1$ an C–2 und die Nitrilgruppe an C–3 oder alternativ der Rest $R^1$ an C–3 und die Nitrilgruppe an C–2 stehen, zwischen C–5 und C–6 eine Einfachbindung vorliegt und die geschlängelten Linien stereoisomere Formen bedeuten.

2. Verfahren zur Herstellung der Verbindungen gemäss Anspruch 1, dadurch gekennzeichnet, dass zunächst aus einem Aldehyd der Formel 7

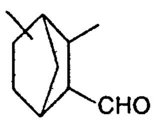

7

das entsprechende Aldoxim 11

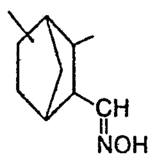

11

dargestellt wird und dass anschliessend das Aldoxim 11 durch Wasserentzug in das Nitril 13

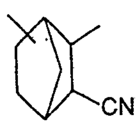

13

überführt wird.

3. Verwendung der Verbindungen gemäss Anspruch 1 als Riechstoffe bzw. Bestandteile von Parfümölen für kosmetische oder technische Konsumgüter.

**Claims**

1. Dimethyl-substituted bycyclo[2.2.1]heptane nitriles of general formula A

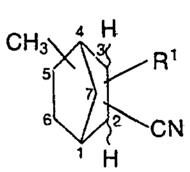

$R^1 = CH_3$

A

wherein

the methyl group occupies the position C–4 or C–5, the residue $R^1$ occupies the position C–2 and the nitrile group the position C–3 or alternatively the residue $R^1$ occupies the position C–3 and the nitrile group the position C–2, a single bond exists between C–5 and C–6, and the wavy lines represent stereoisomeric forms,

2. Process for the preparation of compounds according to claim 1, characterised in that, from an aldehyde of formula 7

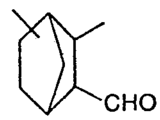

7

the corresponding aldoxime 11

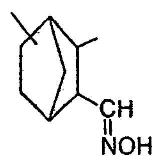

11

is initially produced, and subsequently the aldoxime 11 is converted to the nitrile 13

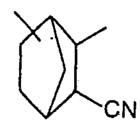

13

by elimination of water.

3. Use of compounds according to claim 1 as scents or components of perfume oils for cosmetic or technical consumer goods.

**Revendications**

1. Bycyclo AD2.2.1 nitrilheptanes à substitution diméthyle de formule générale A

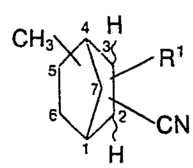

$R^1 = CH_3$

A

dans laquelle

le groupe méthyle se trouve en C–4 ou en C–5, le reste $R^1$ en C–2 et le groupe nitrile en C–3 ou alternativement le reste $R^1$ en C–3 et le groupe nitrile en C–2, une liaison simple existe entre C–5 et C–6 et des lignes sinueuses définissent des formes stéréo-isomères.

2. Procédé de fabrication de composés suivant la revendication 1, caractérisé en ce que, d'abord, à partir d'un aldéhyde de formule 7

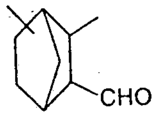

7

on prépare l'aldoxime 11 correspondant

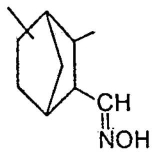

11

et en ce qu'enfin, on transforme l'aldoxime 11 en nitrile 13 par déshydratation

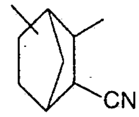

13

3. Utilisation de composés suivant la revendication 1 comme produits odorants ou composants d'essences de parfums pour des produits de consommation cosmétiques ou techniques.

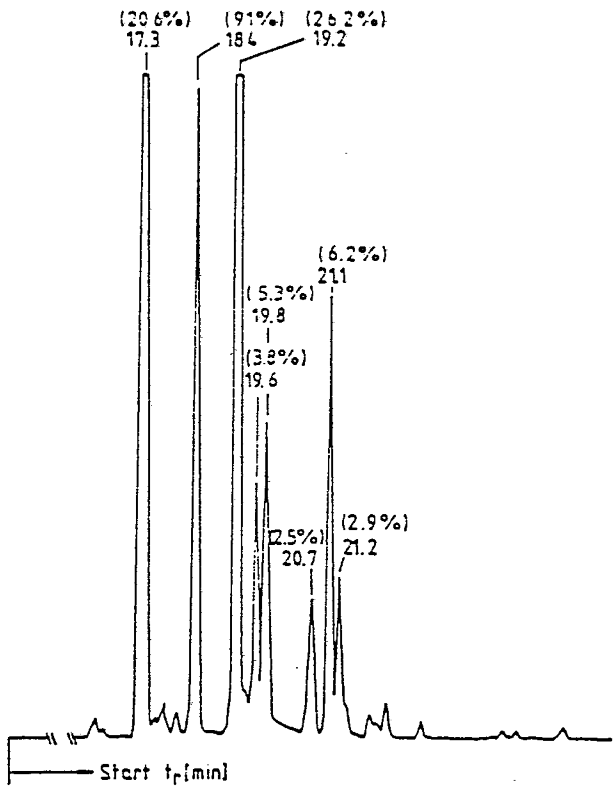


Abb. 1: Gaschromatogramm (25 m FFAP, Temp.-Prog. 60-220°C, 4°C/min) von 7

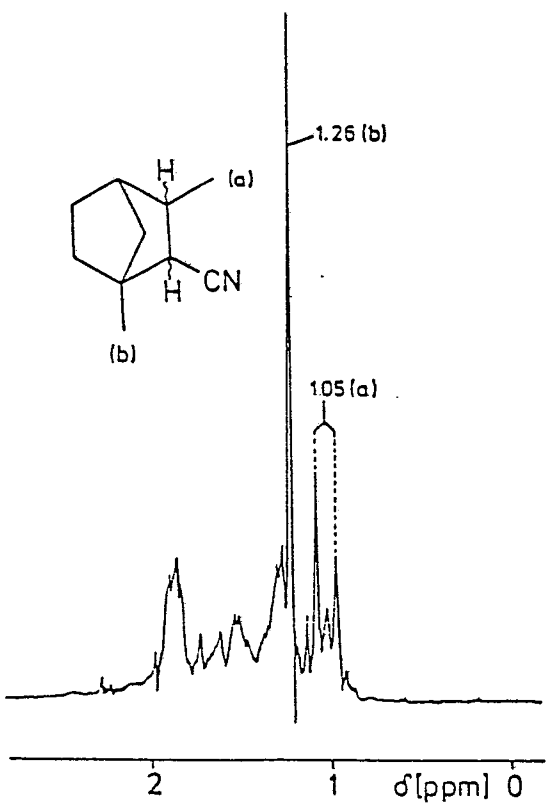


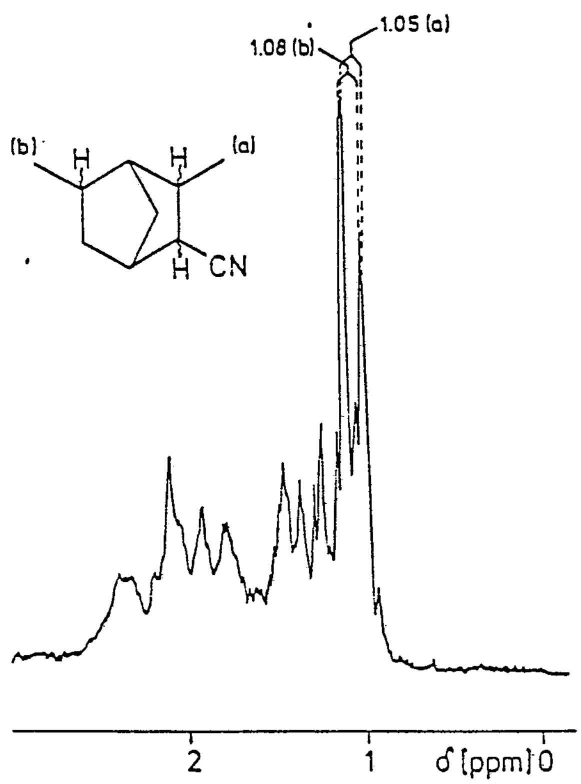


Abb. 2: $^1$H-NMR-Spektrum ($CCl_4$, 60 MHz) von 13 (1. Hauptpeak)

Abb. 3: $^1$H-NMR-Spektrum ($CCl_4$, 60 MHz) von 13 (2. Hauptpeak)